Europäisches Patentamt

European Patent Office

Office européen des brevets

(11) Publication number: **0 287 536**
**A1**

(12) # EUROPEAN PATENT APPLICATION

(21) Application number: 88850087.3

(22) Date of filing: 10.03.88

(51) Int. Cl.⁴: **A 61 K 9/52**
A 61 K 31/49, A 61 K 45/02,
A 61 K 37/02

(30) Priority: 16.03.87 SE 8701077

(43) Date of publication of application:
19.10.88 Bulletin 88/42

(84) Designated Contracting States:
AT BE CH DE ES FR GB IT LI LU NL SE

(71) Applicant: **LEJUS MEDICAL AKTIEBOLAG**
**Taljegardsgatan 3**
**S-431 33 Mölndal (SE)**

(72) Inventor: **Appelgren, Curt Henry**
**PI 1461**
**S-434 00 Kungsbacka (SE)**

**Eskilsson, Eva Christina**
**Körsbärsvägen 15**
**S-435 00 Mölnlycke (SE)**

**Johansson, Sören Ingemar**
**Blacktjärnsgatan 3E**
**S-416 78 Göteborg (SE)**

(74) Representative: **Inger, Lars Ulf Bosson**
**L + U INGER Patentbyra AB Garvaregatan 12**
**S-262 63 Ängelholm (SE)**

The title of the invention has been amended (Guidelines for Examination in the EPO, A-III, 7.3).

(54) **Pharmaceutical quinidine composition.**

(57) The present invention relates to a pharmaceutical composition in a multiple-unit dosage granular form comprising quinidine salt as a therapeutically active compound, and intended to be administered once per 24 hrs, whereby the composition releases quinidine salt in accordance with US Pharmacopea Standards (USP XXI, apparatus 2, 75 rpm) in an artificial gastric juice having pH 1.2 to an extent of less than 30% after 2 hrs of exposure; and releases according to the same Statndard in a phosphate buffer having pH 6.8 to an extent of more than 25%, but less than 90% after 3 hrs of exposure in said phosphate buffer, pH 6.8, (a total exposure of 5 hrs) and releases more than 60% after 8 hrs of exposure (a total of 10 hrs of exposure); and releases less than 100% after 4 hrs of exposure (a total of 6 hrs of exposure) in said phosphate buffer; and releases quinidine salt to an extent of more than 90% after 12 hrs of exposure (a total of 14 hrs of exposure) in said phosphate buffer.

Fig. 6

## Description

<p style="text-align:center"><b>PHARMACEUTICAL COMPOSITION</b></p>

### Technical field

The present invention relates to a pharmaceutical composition in a multiple-unit granular dosage form comprising a quinidine salt as a therapeutically active compound and intended to be administered once per 24 hrs.

The object of the present invention is to obtain a pharmaceutical composition comprising a quinidine salt selected from the group consisting of gluconate, sulphate, and/or hydrogen sulphate, which quinidine salt can be administered once per 24 hrs achieving acceptable therapeutical plasma levels over the whole 24 hrs period.

### Background of the invention

Quinidine is a compound which is used against the indications of relapsing ventricular fibrillation (supporting treatment), extra systoles, and paroxysmal tachycardia.

Quinidine is generally administered in the form of its sulphate or gluconate salts, whereby hitherto known compositions have meant that a number of administrations have to take place every 24 hrs. This is highly depending on the fact that these quinidine salts are very readily soluble in acidic environment, whereby the solubility is drastically reduced at a pH of about 6.5. This means that the solubility in the intestine with its higher pH is small, while the low pH of the stomach provides for a high solubility, which leads to problem in formulating a composition, as one does not want all of the compound present to be dissolved in the stomach with related high plasma levels, which in turn leads to undesired side-effects. The quinidine hydrogen sulphate is an acid salt, which means that it has a different solubility profile than the other two salts mentioned. In order to reach therapeutical levels and effects it is also of importance that large amounts of quinidine are administered per 24 hrs. Thus one wants to administer 800-2000 mg per 24 hrs, normally, however, 800-1200 mg per 24 hrs.

It is known to administer quinidine gluconate as a sustained release preparation (DURA-TABS) but not even this gives a possibility of a once-daily administration, but administration has to take place twice per 24 hrs.

### Description of the present invention

It has now surpringly been shown possible to be able to fulfill the above stated objects and to eliminate hitherto known drawbacks by means of the present invention, which is characterized in that the composition releases, according to the US Pharmacopea Standards (USP XXI (711), apparatus 2, 75 rpm) a quinidine salt in artificial gastric juice at pH 1.2 to a less extent than 30%, preferably less than 20%, more preferably less than 10% after 2 hrs; releases, according to the same standard (USP XXI, apparatus 2, 75 rpm), in a phosphate buffer of pH 6.8 more than 25% and less than 90% after 3hrs of exposure to said buffer, and a total of 5 hrs of exposure for release; more than 60% after 8 hrs of exposure to said buffer, and a total of 10 hrs of exposure for release; less than 100% of release after 4 hrs of exposure to said buffer, and a total of 6 hrs of exposure for release; and more than 90% after 12 hrs of exposure to said buffer, and a total of 14 hrs of exposure for release.

Further characteristics are evident from the accompanying claims.

The Standard used for release is US Pharmacopea Standards (USP XXI (711), apparatus 2, 75 rpm) having a stirring rate of 75 rpm.

The artificial gastric juice used has a pH of 1.2 and consists, in accordance with said Standard, of 175 mls of concentrated HCl (35%), and 50 g of NaCl, diluted to 25000 mls using distilled water.

An acetate buffer used herein having a pH of 5.4 consists of 68.91 g of water free sodium acetate, and 9.61 g of concentrated acetic acid, diluted to 10000 mls using distilled water.

The phosphate buffer used having a pH of 6.8 consists, in accordance with said Standard, of 170.125 g of $KH_2PO_4$, and 23 g of NaOH, diluted to 25000 mls using distilled water.

The invention will be described more in detail with reference to the examples given, however, without being restricted thereto.

### Example 1.

850 g of quinidine gluconate were mixed in dry form with 150 g of microcrystalline cellulose (Avicel PH101), and were then moistened to a moist pulverulent mass using water. The mass was extruded in an extruder (NICASYSTEM), and the extrudate obtained was spheronized in a spheronizer (NICASYSTEM), whereby spherical particles were obtained having a diameter of 0.5 to 1.5 mm. The spheres obtained were then coated in a fluidized bed using a mixture of hydroxypropyl methyl cellulose phtalate (HP55) and ethyl cellulose in the relationship 50:50, dissolved in methylene chloride-ethanol (50:50). The granules were coated with 10% by weight of the ingoing granulate-product weight with said polymer mixture.

### Example 2.

850 g of quinidine sulphate were mixed in dry form with 150 g of microcrystalline cellulose comprising carboxymethylcellulose (Avicel RC 581) and were then moistened to a moist pulverulent mass using water. The mass was extruded in an extruder (NICASYSTEM), and the extrudate obtained was spheronized in a spheronizer (NICASYSTEM), whereby spherical particles were obtained having a diameter of 0.5 to 1.5 mm. The spheres obtained were then coated in a fluidized bed coater using a mixture of EUDRAGIT S100 (methylmethacrylate methylester; 5%), hydroxypropyl methyl cellulose phtalate (HP 55S; 90%) and cetanol (5%). dissolved in methylene chloride-ethanol (50:50). The granules were coated with 8% by weight of the ingoing granulate-product weight with said polymer mixture.

Hydroxypropyl methyl cellulose phtalate and ethyl cellulose can be combined in the relationships 70-35:30-65, normally 60-40:40-60. The amount of coating substance can vary between 2 to 20 % by weight, more preferably between 4 to 12% by weight.

Other coatings than the combination of hydroxypropyl methyl cellulose phtalate and ethyl cellulose, can be anionic polymers having a $pk_a$ of 5.0-6.5, and an insoluble polymer. Insoluble polymers are EUDRAGIT RS, and RL, which are copolymers of polyethyl acrylate (63-65%), methyl methacrylate (31.7-32.3%), and trimethylammonium ethyl methacrylate chloride (2.5-5%), whereby RS has 2.5% of the latter monomer, and RL has 5% of the latter one. Other anionic polymers are cellulose acetate phtalate, EUDRAGIT L, EUDRAGIT S, which later are methyl methacrylate methylesters comprising carboxylic acid groups, and polyvinyl acetate phtalate.

The release profiles of some different compositions having a core of Example 1 above, but being coated with different coating compositions are given in the attached Figures. The release relates to in vitro in artificial gastric juice and phosphate buffer according to USP XXI, apparatus 2, 75 rpm, whereby FIG. 1 shows the result using a coating comprising 50% of each of hydroxypropyl methyl cellulose phtalate and ethyl cellulose, FIG. 2 shows the result using a coating comprising 40% of hydroxypropyl methyl cellulose phtalate and 60% of ethyl cellulose, while FIG. 3 shows the reverse combination having 60% of hydroxypropyl methyl cellulose phtalate and 40% of ethyl cellulose. The hydroxypropyl methyl cellulose phtalate used was HP55.

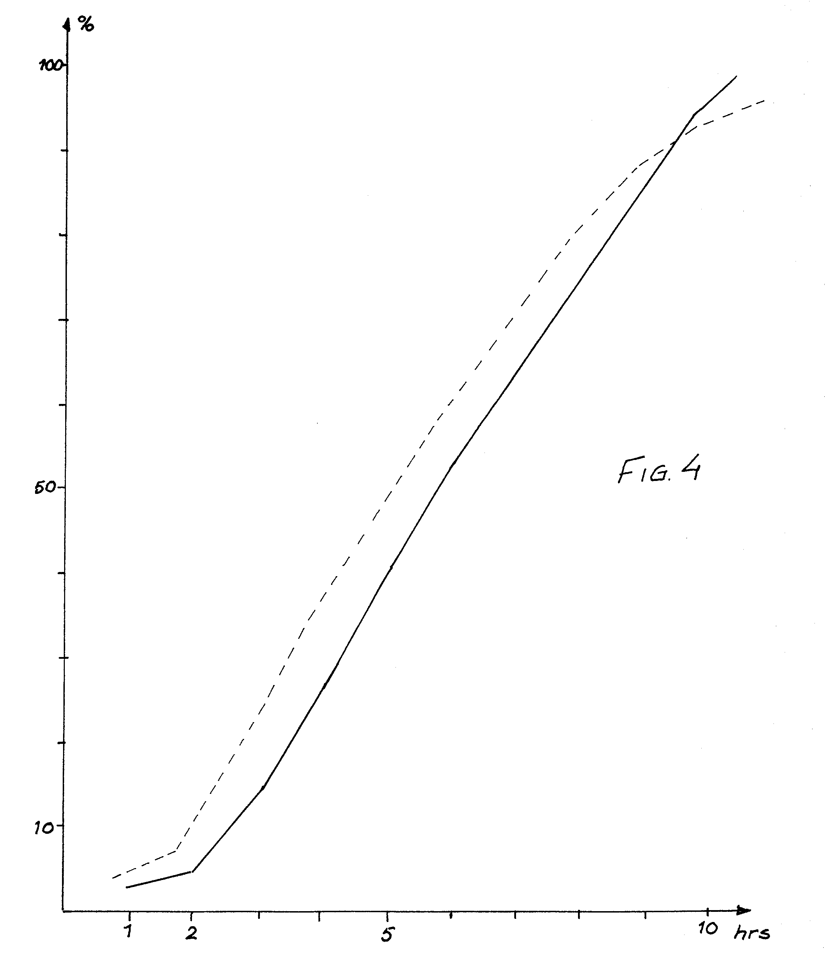

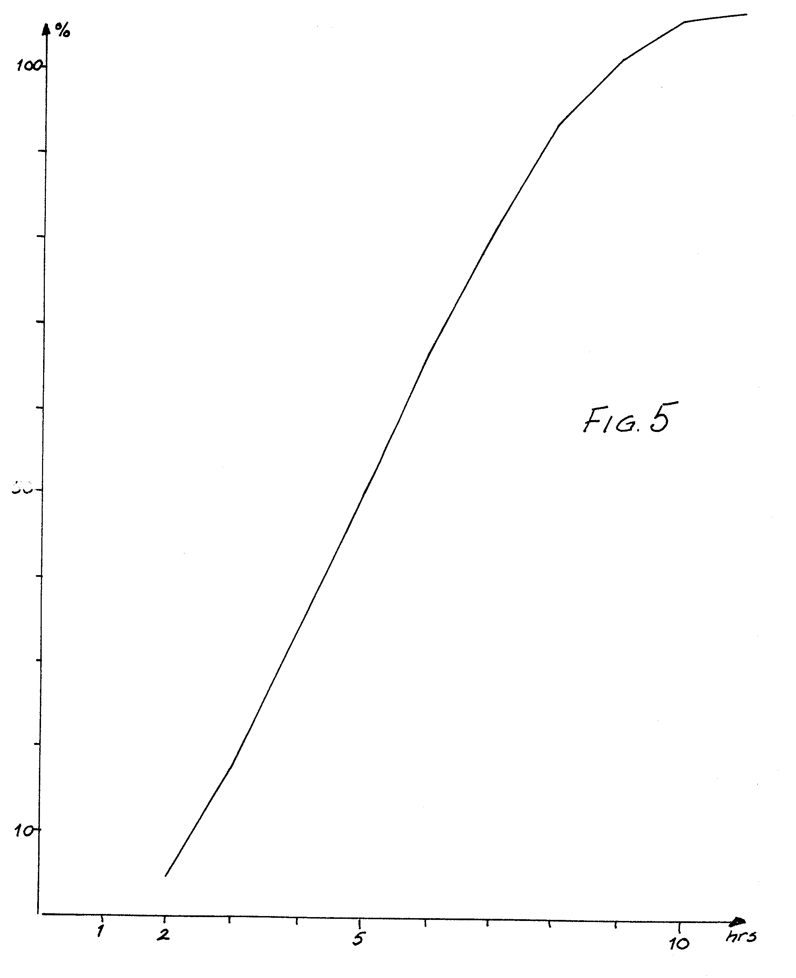

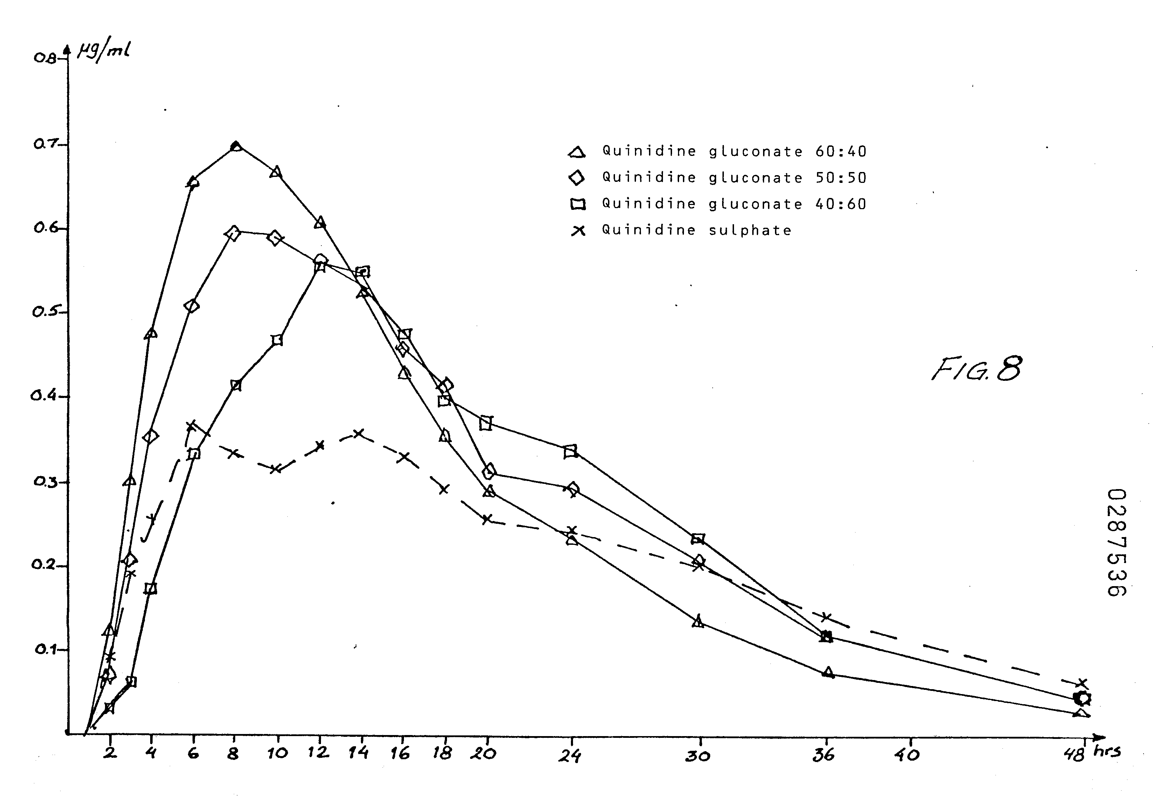

The quinidine sulphate composition of Example 2 prepared having the same in vitro profile as the aforegoing quinidine gluconate composition was tested. The release in vitro is evident from FIG. 4 (USP XXI, apparatus 2, 75 rpm) and FIG. 5 (USP XXI, apparatus 2, 100 rpm). The dotted line gives the release of quinidine gluconate in vitro. Tests carried out shows that the release in vivo of this quinidine sulphate composition does not exhibit the same release as the quinidine gluconate, but gives a bioavailablity which is 60% of that of the quinidine gluconate composition (see FIG. 8).

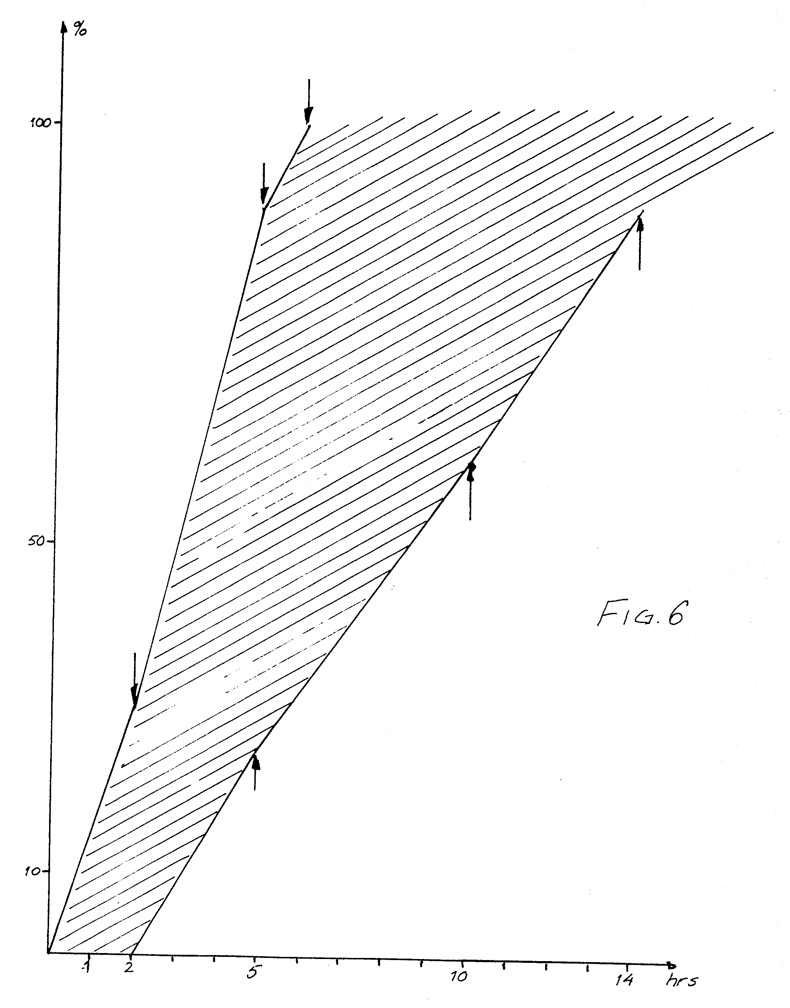

FIG. 6 shows the release profile of the pharmaceutical composition of the present invention according to the above given definition.

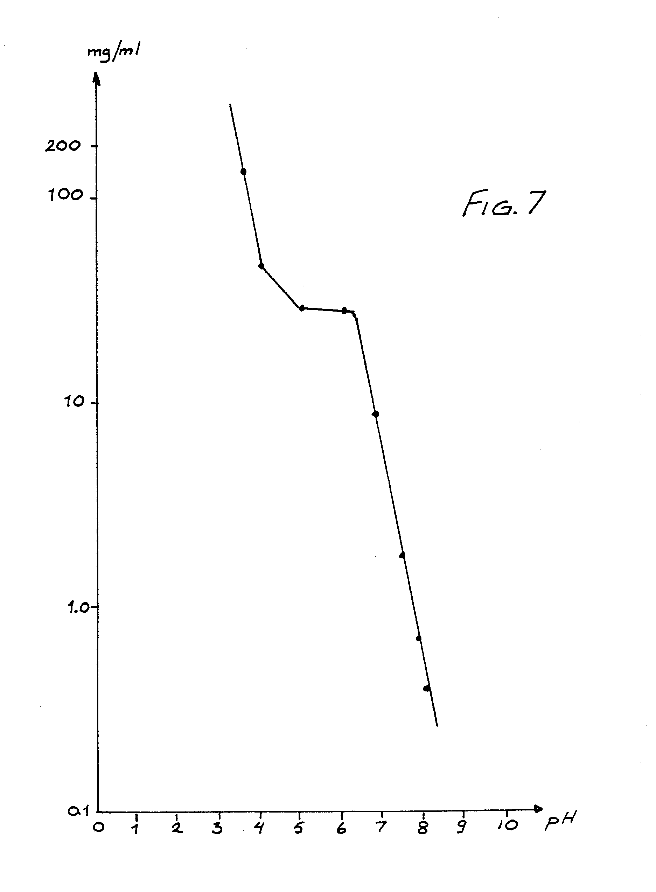

FIG. 7 shows the solubility profile of the quinidine gluconate against pH.

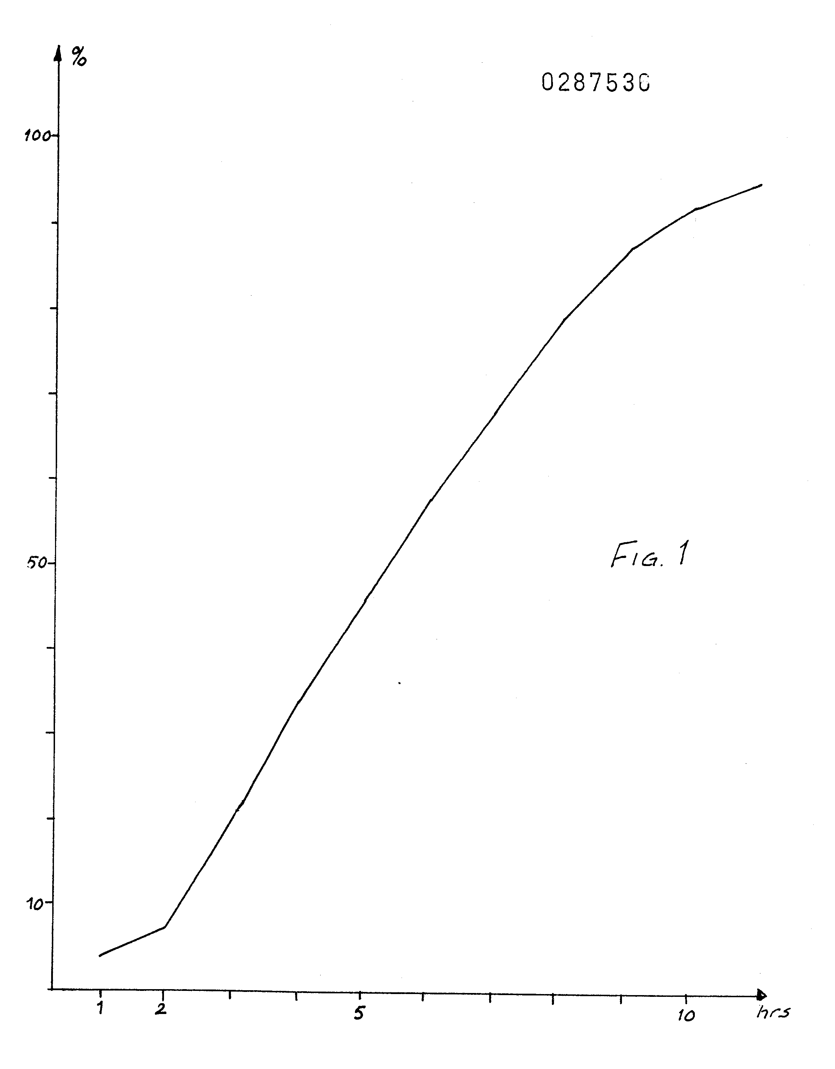

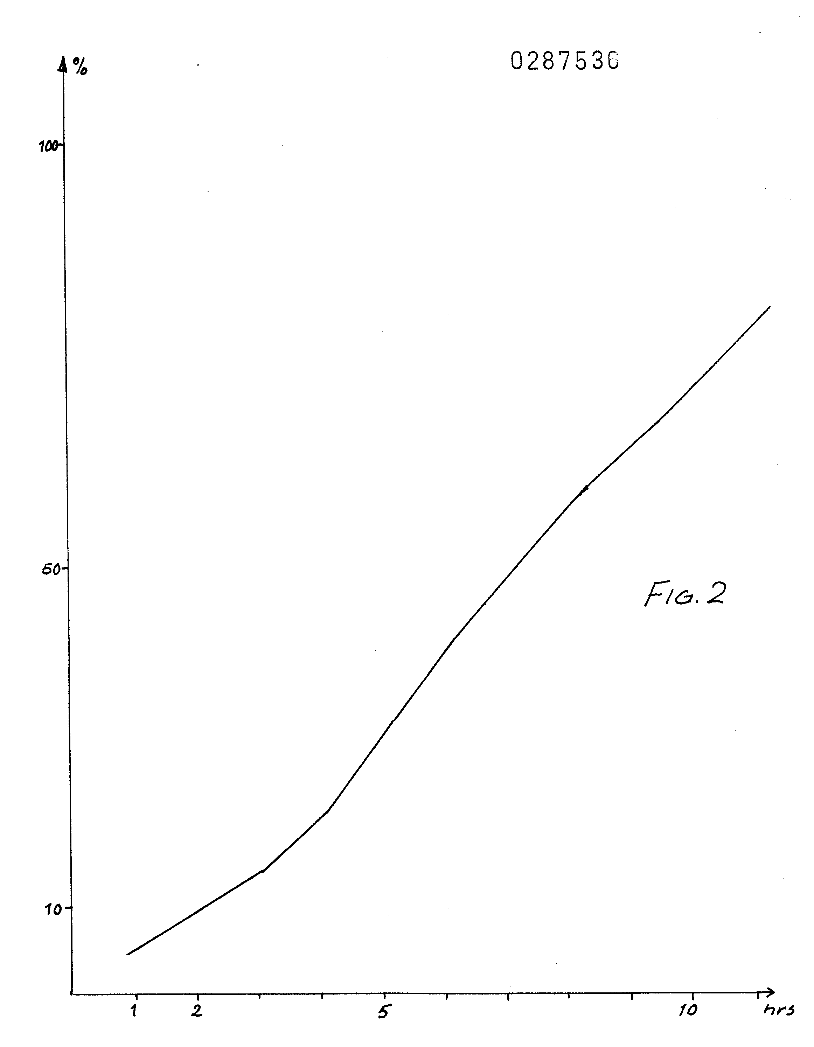

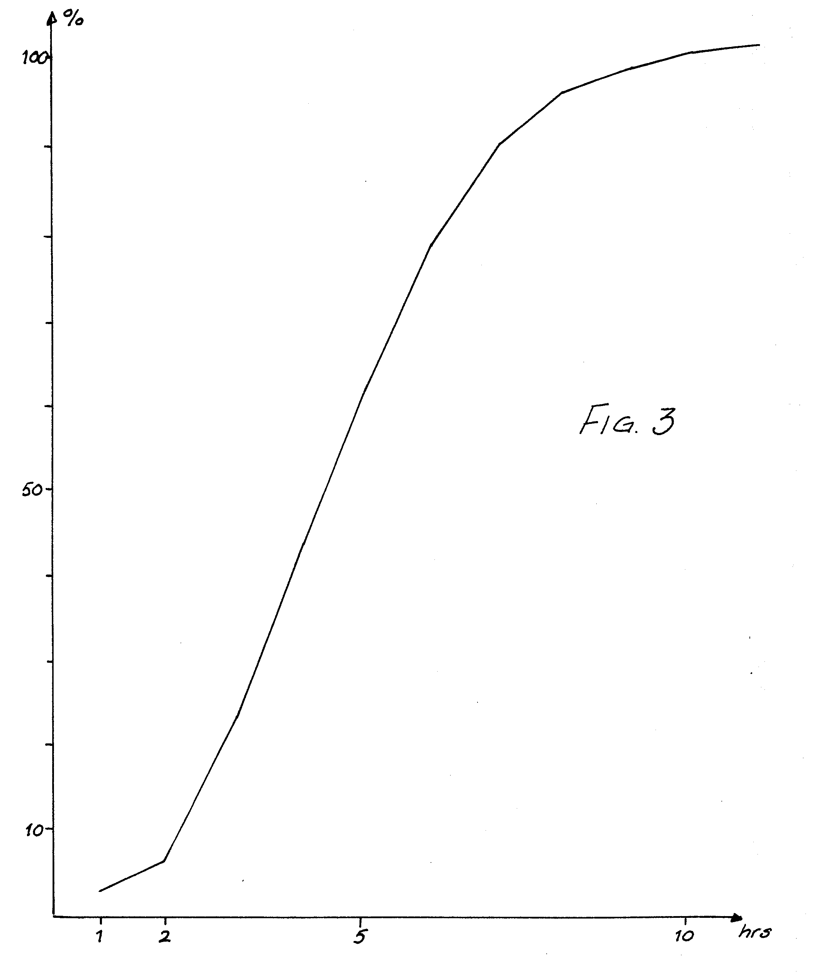

FIG. 8 shows the release in vivo of compositions of the present invention, a quinidine gluconate, and a quinidine sulphate, composition respectively, of above. As evident from FIG. 8 the compositions of the invention comprising quinidine gluconate, FIGS. 1-3 have the same bioavailability, while the composition of the invention comprising quinidine sulphate has a lower bioavailability.

The quinidine gluconate composition above can of course contain a less amount of quinidine gluconate (or other salt) than 85% (Ex. 1), but with regard to the demand for administering large amounts the content should be as large as possible. It should, however, be at least 50% with regard to the volume of administration, and can be up to 95%.

The quinidine salt granules of the invention are packed in a gelatine capsule which dissolves in the gastric juice of the stomach, or in a SACHET-bag, whereby 2000-3000 granules are present in one dose. By means of the gastric peristalsis the stomach will act as a depot which slowly releases granules to the intestinal tract, whereby the granular composition functions as a multiple-unit-dosage formulation, although it for practical reasons is administered in a closed, one-dose unit. The daily dose of quinidine salt is patient dependent, but is, as stated above, normally 800-2000 mg, more normally 800-1200 mg of quinidine gluconate per 24 hrs.

By means of the present invention a reduced release is obtained at low pH values, while at pH values above 6 the release is considerably increased.

Using an acetate buffer, pH 5.4, and consisting of 68.91 g of water free sodium acetate, and 9.61 g of conc. acetic acid which are diluted to 10000 mls using distilled water, no considerably release of quinidine gluconate is obatined, but the release from the composition is still substantially the same as it is in artificial gastric juice.

Quinidine has good and bad absorbers, i.e. different patients absorb quinidine to a different degree, but surprisingly, it has turned out that when using a composition according to the present invention a less divergence is obtained between the patients.

## Claims

1. A pharmaceutical composition in a multiple-unit granular dosage form comprising quinidine salt selected from the group consisting of gluconate, sulphate, and hydrogen sulphate as a therpeutically active component, and intended to be administered once per 24 hrs, **characterized** in that the composition releases quinidine salt in accordance with US Pharmacopea Standards (USP XXI, apparatus 2, 75 rpm) in an artificial gastric juice having pH 1.2 to an extent of less than 30% after 2 hrs; and releases according to the same Standard in a phosphate buffer having pH 6.8 to an extent of more than 25% but less than 90% after 3 hrs of exposure in said phosphate buffer, pH 6.8, (a total exposure of 5 hrs) and releases more than 60% after 8 hrs of exposure (a total of 10 hrs of exposure); and releases less than 100% after 4 hrs of exposure (a total of 6 hrs of exposure) in said phosphate buffer; and releases quinidine salt to an extent of more than 90% after 12 hrs of exposure (a total of 14 hrs of exposure) in said phosphate buffer.

2. Composition according to claim 1, **characterized** in that the release in artificial gastric juice, pH 1.2, is less than 20% after hrs of exposure.

3. Composition according to claims 1-2, **characterized** in that the release in artificial gastric juice, pH 1.2, is less than 10% after 2hrs of exposure.

4. Composition according to claims 1-3, **characterized** in that it comprises a core having a content of 50-95% by weight of quinidine gluconate coated with 2-20% by weight of hydroxypropyl methyl cellulose phtalate, and ethyl cellulose in a mixture having the relationship 70-35:30-65.

5. Composition according to claim 4, **characterized** in that the relationship is 60-40:40-60.

6. Composition according to claims 4-5, **characterized** in that the hydroxypropyl methyl cellulose phtalate and ethyl cellulose has been added in an amount of 4-12% by weight.

7. Composition according to claim 6, **characterized** in that the mixture has been added in an amount of 7-10% by weight of the weight of the granules.

8. Composition according to claims 4-7, **characterized** in that ingoing hydroxypropyl methyl cellulose phtalate has a $pk_a$ of 5.0-6.5, preferably 5.5.

9. Composition according to one or more of the preceeding claims, **characterized** in that ingoing hydroxypropyl methyl cellulosa phtalate has been exchanged for cellulose acetate phtalate, methylmethacrylate methylester (EUDRAGIT L, and S), and/or polyvinyl acetate phtalate.

10. Composition according to one or more of the preceeding claims, **characterized** in that ingoing ethyl cellulose has been exchanged for a copolymer of polyethyl acrylate (63-65%), methyl methacrylate (31.7-32.3%), and trimethylammonium ethyl methacrylate chloride (2.5-5%) (EUDRAGIT RS, and RL).

11. Composition according to claims 1-3, **characterized** in that it comprises a core having a content of 50-95% by weight of quinidine sulphate coated with 2-20% by weight of a mixture of methyl methacrylate methylester (5%), hydroxypropyl methyl cellulose phtalate (90%), and cetanol (5%).

12. Composition according to claim 11, **characterized** in that ingoing hydroxypropyl methyl cellulose phtalate has a $pk_a$ of 5.0-6.5, preferably 5.5.

0287536

Fig. 1

0287536

%

100

50

FIG. 2

10

1   2        5                    10      hrs

0287536

FIG. 3

0287536

FIG. 4

0287536

FIG. 5

FIG. 6

0287536

FIG. 7

FIG. 8

Legend:
- △ Quinidine gluconate 60:40
- ◇ Quinidine gluconate 50:50
- ⊡ Quinidine gluconate 40:60
- ✕ Quinidine sulphate

0287536

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 400 295 (KEY PHARMACEUTICALS INC.) * Page 2, line 25 - page 3; claims * | 1-3 | A 61 K 9/52 A 61 K 31/49 |
| Y | | 4-12 | |
| Y | EP-A-0 153 105 (A/S ALFRED BENZON) * Page 15, lines 18-19; claims 6,15 * | 1-12 | |
| Y | FR-A-2 237 620 (BYK GULDEN LOMBERG) * Page 20, example 2 * | 1-12 | |
| Y | EP-A-0 040 590 (AKTIEBOLAGET HÄSSLE) * Page 11, example 3 * | 1-12 | |
| X | EP-A-0 148 811 (LEJUS MEDICAL A.B.) * Page 7, examples 1,2 * | 1-3 | |
| Y | | 4-12 | |

TECHNICAL FIELDS SEARCHED (Int. Cl.4)

A 61 K 9/00
A 61 K 31/00

The present search report has been drawn up for all claims

| Place of search | Date of completion of the search | Examiner |
|---|---|---|
| THE HAGUE | 15-07-1987 | GERLI P.F.M. |

EPO FORM 1503 03.82 (P0401)